Europäisches Patentamt

European Patent Office

Office européen des brevets

(19)

(11) Publication number: 0 348 967
A2

## (12) EUROPEAN PATENT APPLICATION

(21) Application number: 89111831.7

(22) Date of filing: 29.06.89

(51) Int. Cl.⁴: C07K 7/00 , A61K 37/02

Claims for the following Contracting States: ES + GR.

(30) Priority: 01.07.88 US 214228
09.12.88 US 282795

(43) Date of publication of application:
03.01.90 Bulletin 90/01

(84) Designated Contracting States:
AT BE CH DE ES FR GB GR IT LI LU NL SE

(71) Applicant: MERRELL DOW
PHARMACEUTICALS INC.
2110 East Galbraith Road
Cincinnati Ohio 45215-6300(US)

(72) Inventor: McLean, Larry R.
37 W. Charlotte Avenue
Cincinnati Ohio 45215(US)
Inventor: Krstenansky, John L.
3749 Ault Park
Cincinnati Ohio 45208(US)

(74) Representative: Vossius & Partner
Siebertstrasse 4 P.O. Box 86 07 67
D-8000 München 86(DE)

(54) Synthetic lung surfactant preparation comprising lipids and polypeptides.

(57) Synthetic pulmonary surfactant consisting of a complex of a polypeptide having alpha-helical structure and a lipid consisting of one or more of the lipids associated with natural pulmonary surfactant were prepared and evaluated in animal failing lung models.

FIGURE 1

# SYNTHETIC LUNG SURFACTANT PREPARATION COMPRISING LIPIDS AND POLYPEPTIDES

This invention relates to the synthesis of a series of polypeptides whose sequence is unrelated to the sequences of proteins in mammalian lung surfactant, the preparation of mixtures of these polypeptides with lipids, the method for production of the same and pharmaceutical compositions which are effective in the treatment of mammalian respiratory distress syndrome.

Infantile respiratory distress syndrome (RDS) is a leading cause of death in the first 28 days of life. It strikes 1 in 100 babies worldwide and about 10 percent die. The syndrome rarely occurs in term infants but is generally associated with immaturity and in low-birth weight (under 2 kg). Adult RDS shows similar clinical characteristics and pathophysiology as the infantile disease and is managed in the intensive care facility in a similar fashion. The adult disease has diverse etiologies and results from lung insults such as diffuse infections, aspiration of the gastric contents or water, inhalation of irritants and toxins and pulmonary edema arising from such sources as narcotic overdose. RDS is correlated with an absence or dysfunction of the lung surfactant which coats the alveoli of the lungs where gas exchange occurs.

Lung surfactant is composed primarily of lipid (90%) and contains a minor component of various proteins (10%). The primary lipid type is phospholipid which constitutes 97% of the lipid. 80% of this phospholipid is phosphatidylcholine (PC) and 10% is phosphatidyglycerol (PG). 70-80% of the phospholipid acyl chains are saturated and 85-90% of these are 16-carbons in length. The major lipid is dipalmitoylphosphatidylcholine (DPPC). The protein component of lung surfactant is also heterogeneous but more poorly characterized. The major protein is ~32kd and its sequence has been predicted from its cDNA (White et al. (1985) Nature 317, 361-363) and it has been cloned (Floros, et al. Journal of Biological Chemistry (1985) 260, 495-500). Minor proteins of lower molecular weight have also been isolated and several sequences have been reported (Warr, et al. (1987) Proceedings of the National Academy of Sciences USA 82, 7915-7919; Glasser, et al. (1988) Journal of Biological Chemistry 263, 9; Johansson, et al. (1988) FEBS Letters. 232, 61-64; Glasser, et al. (1987) Proceedings of the National Academy of Sciences USA 84, 4007; Revak, et al. (1988) Journal of Clinical Investigation 81, 826-833).

Infantile RDS has been treated with animal lung lavages (Smyth, et al. (1983) Pediatrics 71, 913-917; McCord, et al. (1988) Arch. Dis. Child. 63, 10-16) and human lung surfactant obtained from human amniotic fluid (Hallman, et al. (1983) Pediatrics 71, 473-482; (1985) J. Pediatr. 106, 963-969; Merritt, et al. (1986) N. Engl. J. Med. 13, 785-790) with considerable success. Cow lung lavages extracted with organic solvents and mixed with synthetic lipids are also effective (Fujiwara, et al. (1980) Lancet 1, 55-59; Noach, et al. (1986) Eur. J. Respir. Dis. 69, 321-335; Kwong, et al. (1985) Pediatrics 76, 585-592; Enhorning, et al., (1985) Pediatrics 76, 145-153; Shapiro, et al. (1985) Pediatrics 76, 593-599; Gitlin, et al. (1987) Pediatrics 79, 31-37; Raju, et al. (1987) Lancet 1, 651-656; Halliday, et al (1984) Lancet 1, 476-478). However, pure synthetic mixtures have not consistently been shown to be active (Morley, et al., (1981) Lancet 1, 64-68; Ten Centre Study Group (1987) Br. Med. J. 294, 991-996; Milner, et al. (1983) Arch. Dis. Child. 58, 458-460; Milner, et al. (1983, ibid 59, 369-371; and Wilkinson, et al. (1985) Lancet 2, 287-291). We are aware of only a single clinical study using surfactants in adult RDS, (Lochman, et al., (1988) Adv. Exp. Med. Biol. 222, 511-517), and the potential value of such therapy has been indicated in the literature (van Golde, et al. Physiol. Rev. 68, 374-455).

The preferred lung surfactant preparation consists of completely defined components. As only limited success with pure lipid mixtures has been reported, it appears that a protein component of lung surfactant is required. One approach is to use genetically engineered proteins with sequences based on the amino acid sequences predicted from the cDNA of the lung surfactant proteins. An alternative method, which is the subject of the present invention is to synthesize peptides with physical properties suited to the function of lung surfactant when in combination with defined lipid components, but with sequences unrelated to the sequences of proteins isolated from lung surfactant.

The present invention is broadly concerned with synthetic lung surfactant compositions and more specifically with surfactant compositions comprising a mixture of synthetic polypeptides and lipids effective in treating RDS. The synthetic polypeptides have unique sequences which are not found in the sequences of the mammalian proteins isolated from lung lavages. The polypeptides may be used singly in mixtures with lipid or in combination in mixtures of lipid. The polypeptide comprises a minor component of the surfactant mixture.

The chemical composition of the present invention is novel with regard to the peptide component and the combination of such peptides with lipids in a synthetic lung surfactant which is medicinally effective in the treatment of respiratory distress syndrome. The composition of the present invention may be prepared in high purity and in a standardized fashion as it is a defined mixture of synthetic components. Also, the components are not derived from animal sources which minimizes the risk of contamination by viruses and bacteria. Also, the components are biological in type and metabolic pathways are available in mammals for the eventual elimination of the components of the mixture. Also, no nonbiological molecules are introduced.

In the Drawings:

FIG. 1 is a pressure-volume curve for References I and II in the adult rat lung model.

FIG. 2 is a pressure-volume curve showing the effectiveness of Examples 1 and 2 in the adult rat lung model.

The present invention relates to synthetic lung surfactants consisting of a complex of a polypeptide and lipids wherein the polypeptide has the following formula 1

$$X-Y-Z-Y'-Q \qquad 1$$

wherein X is hydrogen, a $C_{(1-5)}$alkyl group, or a $C_{(2-10)}$acyl group;

Y and $Y'$ are each independently a bond or $-(Ser)_n$-where n is an integer of from 1 to 3;

Q is an hydroxy, amino, alkylamino or alkoxy group;

Z is a peptide residue of from 8 to 25 amino acids consisting of a fragment of the oligomer having the sequence

$-A_1-A_2-A_3-A_4-A_5-A_6-A_7-A_8-A_9-A_{10}-A_{11}-A_1'-A_2'-A_3'-A_4'-A_5'-A_6'-A_7'-A_8'-A_9'-A_{10}'-A_{11}'-A_1''-A_2''-A_3''-A_4''-A_5''-A_6''-A_7''-A_8''-A_9''-A_{10}''-A_{11}''-A_1'''-A_2'''-$

and which may begin with any one of the amino acids residues designated $A_1 - A_{11}$ wherein

$A_1$, $A_1'$, $A_1''$, $A_1'''$, $A_4$, $A_4'$, $A_4''$, $A_8$, $A_8'$, and $A_8''$ are each independently selected from the group of hydrophilic amino acids consisting of Glu, Asp, Ala, Gln, Asn, Gly, Ser, Thr, Lys, Arg, Orn, and hArg;

$A_2$, $A_2'$, $A_2''$, $A_2'''$, $A_3$, $A_3'$, $A_3''$, $A_6$, $A_6'$, $A_6''$, $A_7$, $A_7'$, $A_7''$, $A_{10}$, $A_{10}'$, and $A_{10}''$ are each independently selected from the group of lipophilic amino acids consisting of Leu, Nle, Met, Ala, Val, Phe, Nva, Ile and Tyr;

$A_5$, $A_5'$, $A_5''$, $A_{11}$, $A_{11}'$, and $A_{11}''$ are each independently selected from the group of basic amino acids consisting of Lys, Orn, Arg, hArg;

$A_9$, $A_9'$, $A_9''$ are each independently selected from the group of lipophilic neutral or basic amino acids consisting of Leu, Nle, Met, Ala, Val, Phe, Nva, Ile, Tyr, Thr, Ser, Gln, Asn, Gly, Lys, Arg, hArg, Trp, Orn, Trp(For),

or a phamaceutically acceptable salt thereof,

and wherein the lipid is comprised of one or more of the type associated with natural pulmonary surfactant.

These polypeptide-lipid complexes and their pharmaceutical compositions are useful in treating mammalian respiratory distress syndrome.

The following common abbreviations of the naturally occurring amino acids are used throughout this specification:

Ala or A - alanine

Val or V - valine

Leu or L - leucine

Ile or I - isoleucine

Phe or F - phenylalanine

Trp or W - tryptophan

Met or M - methionine

Ser or S - serine

Tyr or Y - tyrosine

Asp or D - aspartic acid

Glu or E - glutamic acid

Lys or K - lysine

Arg or R - arginine

Nle - norleucine

Orn - ornithine

hArg - homoarginine

Nva - norvaline

Trp(For) - $N^{in}$-formyl-Trp

The natural amino acids, with the exception of glycine, contain a chiral carbon atom. Unless otherwise specifically indicated, the optically active amino acids, referred to herein, are of the L-configuration. As is customary, the structure of peptides written out herein is such that the amino terminus is on the left side of the chain and the carboxy terminus is on the right side of the chain.

Certain polypeptides of formula 1 are preferred in the method of treating respiratory distress syndrome of this invention. Those polypeptides of formula 1 wherein at least one of $A_1$, $A_1{}'$, $A_1{}''$, and $A_1{}'''$ is a Glu; wherein at least one of $A_4$, $A_4{}'$, and $A_4{}''$ is a Glu; and wherein at least one of $A_8$, $A_8{}'$, and $A_8{}''$ is a Glu are preferred. Also those polypeptides of formula 1 wherein at least one of $A_2$, $A_2{}'$, $A_2{}''$, and $A_2{}'''$ is a Leu; wherein at least one of $A_3$, $A_3{}'$, and $A_3{}''$ is a Leu; wherein at least one of $A_6$, $A_6{}'$, and $A_6{}''$ is a Leu; wherein at least one of $A_7$, $A_7{}'$, and $A_7{}''$ is a Leu; and wherein at least one of $A_{10}$, $A_{10}{}'$, and $A_{10}{}''$ is a Leu are preferred. Also preferred are those polypeptides of formula 1 wherein at least one of $A_5$, $A_5{}'$, and $A_5{}''$ is a Lys; wherein at least one of $A_9$, $A_9{}'$, and $A_9{}''$ is a Phe; and wherein at least one of $A_{11}$, $A_{11}{}'$, and $A_{11}{}''$ is a Lys. Additionally applicants prefer those polypeptides of formula 1 werein Q is an amino group.

The polypeptides of this invention can be prepared by a variety of procedures readily known to those skilled in the art. Such procedures include the solid phase sequential and block synthesis, gene cloning and combinations of these techniques. The solid phase sequential procedure can be performed using established automated methods such as by use of the ABI peptide synthesizer. In this procedure an $\alpha$-amino protected amino acid is bound to a resin support. The resin support employed can be any suitable resin conventionally employed in the art for the solid phase preparation of polypeptides, preferably polystyrene which has been cross-linked with from 0.5 to about 3 percent divinyl benzene, which has been either chloromethylated or hydroxymethylated to provide sites for ester formation with the initially introduced $\alpha$-amino protected amino acid.

An example of a hydroxymethyl resin is described by Bodanszky, et al., Chem. Ind. (London) 38, 1597-98 (1966). A chloromethylated resin is commercially available from Bio Rad Laboratories, Richmond, California and the preparation of such a resin is described by Stewart, et al., "Solid Phase Peptide Synthesis" (Freeman & Co., San Francisco 1969), Chapter 1, pp. 1-6. The protected amino acid can be bound to the resin by the procedure of Gisin, Helv. Chem. Acta, 56, 1476 (1973). Many resin bound, protected amino acids are commercially available. As an example, to prepare a polypeptide of this invention wherein the carboxy terminal end is a Thr residue, a tert-butyloxycarbonyl (Boc) protected Thr bound to a benzylated, hydroxymethylated phenylacetamidomethyl (PAM) resin can be used and is commercially available.

Following the coupling of the $\alpha$-amino protected amino acid to the resin support, the protecting group is removed using any suitable procedure such as by using trifluoroacetic acid in methylene chloride, trifluoroacetic acid alone, or HCL in dioxane. The deprotection is carried out at a temperature between $0°$ C and room temperature. Other standard cleaving reagents and conditions for removal of specific $\alpha$-amino protecting groups may be used. After removal of the $\alpha$-amino protecting group the other amino protected amino acids are coupled step-wise in the desired order. Alternatively, multiple amino acid groups may be coupled by the solution method prior to coupling with the resin supported amino acid sequence.

The $\alpha$-amino protecting group employed with each amino acid introduced into the polypeptide sequence may be any such protecting group known to the art. Among the classes of $\alpha$-amino protecting groups contemplated are (1) acyl type protecting groups such as: formyl, trifluoroacetyl, phthalyl, toluenesulfonyl (tosyl), benzenesulfonyl, nitrophenylsulfenyl, tritylsulfenyl, o-nitrophenoxyacetyl and $\gamma$-chlorobutyryl; (2) aromatic urethan type protecting groups such as benzyloxycarbonyl and substituted benzyloxycarbonyl such as p-chlorobenzyloxycarbonyl, p-nitrobenzyloxycarbonyl, p-bromobenzyloxycarbonyl, p-methoxybenzyloxycarbonyl, 1-(p-biphenyl)-1-methylethoxycarbonyl, $\alpha,\alpha$-dimethyl-3,5-dimethoxybenzyloxycarbonyl and benzhydryloxycarbonyl; (3) aliphatic urethan protecting groups such as tert-butyloxycarbonyl (Boc), diisopropylmethoxycarbonyl, isopropyloxycarbonyl, ethoxycarbonyl and allyloxycarbonyl; (4) cycloalkyl urethan type protecting groups such as cyclopentyloxycarbonyl, adamantyloxycarbonyl and cyclohexyloxycarbonyl; (5) thio urethan type protecting groups such as phenylthiocarbonyl; (6) alkyl type protecting groups such as triphenylmethyl (trityl) and benzyl; (7) trialkylsilane groups such as trimethylsilane. The preferred $\alpha$-amino protecting group is tert-butyloxycarbonyl.

The selection of an appropriate coupling reagent is within the skill of the art. A particularly suitable coupling reagent where the amino acid to be added is Gln, Asp or Arg is N,N$'$-diisopropylcarbodiimide and 1-hydroxybenzotriazole. The use of these reagents prevents nitrile and lactam formation. Other coupling agents are (1) carbodiimides (e.g., N,N$'$- dicyclohexylcarbodiimide and N-ethyl-N$'$-($\gamma$-dimethylaminopropylcarbodiimide); (2) cyanamides (e.g., N,N-dibenzylcyanamide); (3) ketenimines; (4) isoxazolium salts (e.g., N-ethyl-5-phenyl isoxazolium-3$'$-sulfonate; (5) monocyclic nitrogen containing heterocyclic amides of aromatic character containing one through four nitrogens in the ring such as imidazolides, pyrazolides and 1,2,4-triazolides. Specific heterocyclic amides that are useful include N,N$'$-carbonyldiimidazole and N,N$'$-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., (Boc-Arg)$_2$-O) and (8) nitrogen containing heterocyclic compounds having a hydroxy group on one ring nitrogen (e.g.,

4

N-hydroxyphthalimide, N-hydroxysuccinimide and 1-hydroxybenzotriazole). Other activating reagents and their use in peptide coupling are described by Kapoor, J. Pharm. Sci., 59, pp. 1-27 (1970). Applicant prefers the use of the symmetrical anhydride as a coupling reagent.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser, et al., Analyt. Biochem. 34, 595 (1970).

After the desired amino acid sequence has been obtained, the peptide is removed from the resin. This can be done by methanolysis such as by treatment of the resin bound polypeptide with a solution of dimethyl sulfide, p-cresol and thiocresol in dilute aqueous hydrofluoric acid.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed by cleavage during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxycarbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The preferred protecting group is benzyl.

These groups are removed by procedures well known in the art. Typically protecting group removal is done after the peptide chain synthesis is complete but the protecting groups can be removed at any other appropriate time.

The polypeptides of Formula 1 can form pharmaceutically acceptable salts with any non toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-ethenamine, N,N'-dibenzylethylenediamine, dihydroabietylamine, N-(lower)alkylpiperidine, and any other suitable amine.

The phospholipids of the protein-phospholipid complexes of this invention can be any phospholipid and this term as used herein includes the phosphoglycerides and the sphingolipids. Phosphoglycerides are those di-fatty acid esters of glycerol in which the remaining hydroxy group, a terminal hydroxy group, of the gylcerol moiety forms an ester with phosphoric acid. Commonly the phosphoric acid moiety of the phosphoglycerides forms a second ester with an alcohol such as ethanolamine, serine, choline, or glycerol. Sphingolipids are those mono-fatty acid esters of sphingosine or dihydrosphingosine in which the hydroxy group at the 1-position forms an ester with the choline ester of phosphoric acid. The preferred lipids of the protein-phospholipid complexes of this invention comprise dipalmitoylphosphatidylcholine (DPPC), phosphatidylcholine molecules containing acyl chains of other lengths and degrees of saturation (PC), cardiolipin (CL), phosphatidylglycerols (PG), phosphatidylserines (PS), fatty acids (FA), and triacylglycerols (TG). DPPC comprises the major component of the lung surfactant mixture while PC, CL, PG, PS, FA, and TG comprise minor components. Suitable fatty acids for use in the phospholipids of this invention are long chain carboxylic acids (generally having eight or more carbon atoms), typically unbranched. The fatty acids can be either saturated or unsaturated. Representative fatty acids are lauric, myristic, palmitic, and oleic acids.

Pharmaceutical preparations of the protein-phospholipid complexes of this invention can be prepared as a dry mixture or in an aqueous suspension, in some instances containing small amounts of organic solvents, such as, for example, ethanol or trifluoroethanol, detergents, such as, for example, sodium dodecyl sulfate or sodium deoxycholate, salts, such as calcium chloride or sodium chloride, carbohydrates, such as glucose, dextrose or mannitol, and amino acids, such as glycine and alanine. Where the pharmaceutical composition is made into liquid form, stabilizers, preservatives, osmotic pressure regulators, buffering agents, and suspending agents of the liquid may be added. If desired, suitable germicides may also be added. The pH of the aqueous suspension may vary between 2 and 10 and may be adjusted with acids and bases, such as, for example, hydrochloric acid, sodium phosphate, or sodium hydroxide. The dry mixture may be reconstituted in an aqueous solution containing pharmaceutically acceptable salts, organic solvents, and detergents. The aqueous preparation may be dialyzed, filtered, or chromatographed to exchange the suspending medium with a pharmaceutically acceptable medium prior to use. The preparation may be administered as a dry powder, an aqueous suspension, or as an aerosol directly into the lungs of the distressed subject. The pharmaceutical composition of the present invention may be charged in hermetically sealed containers such as vials and ampules and be preserved sterilely. The composition may be stored in a vial or ampule separately from a vial or ampule containing the suspension buffer and the dry or hydrated composition may be mixed with the suspension buffer prior to use.

Lipid constitutes from 50 to 99.9% of the lung surfactant preparation. Suitable lipids include DPPC, PC, CL, PG, PS, FA, and TG. DPPC comprises the major lipid species and is present in concentrations of 60 to 100% of the total lipid weight. The remaining lipids are present in minor concentrations. PC, CL, PG and PS may comprise up to 30% of the lipids, and FA and TG may comprise up to 10% of the lipid weight. The fatty acyl chains of the minor lipid components may be saturated or unsaturated and of any chain length. Chain lengths of 12 to 16 carbon atoms and up to 2 unsaturated bonds are preferred. The preferred lipid composition is 85-100% DPPC plus 0-15% of PG.

The lipid components of the synthetic lung surfactant are commonly found in mammalian lung surfactant and are available from common industrial sources in high purity. The polypeptide components are prepared by solid-phase peptide synthesis by methods familiar to those skilled in the art. Sequences similar to the sequences of the polypeptides in this invention have been used as models for the lipid-binding region of plasma apolipoproteins, but have not been used in synthetic lung surfactant preparations. Mixtures of the lipids of the invention with proteins isolated from mammalian lung lavage have been shown to be effective in treating neonatal RDS. However, mixtures of these lipids with synthetic peptides in lung surfactant preparations have not been reported.

Lipids are mixed in a volatile organic solvent or mixtures of solvents, such as mixtures of chloroform and methanol. The organic solvent is removed by evaporation under nitrogen, argon, or under vacuum. An aqueous solution which may contain organic and inorganic acids, bases, and salts, and saccharides such as dextrose is added to the dry lipid mixture to attain a final concentration of 0.1 to 100 mg of DPPC per ml. In general, it is preferable, but not necessary to warm the mixture to 35-50°C, mix vigorously, and incubate for up to 2 hours at 25-50°C. Then, peptide or a mixture of peptides is added as a dry powder or suspended in an aqueous solution in some cases containing a suitable organic solvent, such as ethanol or trifluorethanol, or a denaturing agent, such as guanidinium hydrochloride or urea, which improves the solubility of the peptide in the aqueous suspension. Association of peptide and lipid may be promoted at a particular pH, thus the pH of the aqueous solution may vary from 2 to 10. The preferred method for mixing peptide and lipid is to add dry peptide to lipid in water at 45-50°C and to mix by bath ultrasonication at 45-50°C for 30-90 minutes, then freeze-dry and store at -20°C.

Lipids are mixed with a suitable detergent such as octylglucoside or sodium deoxycholate at a weight ratio of from 1 to 20 parts of detergent per part of DPPC in water, an aqueous buffer, or saline solution at concentrations from 1 to 100 mg DPPC/ml. Then, peptide is added as a dry powder or suspended in an aqueous solution with or without an organic solvent, denaturing agent, or detergent. The mixture is then dialyzed, filtered, centrifuged or chromatographed to remove the detergent.

Alternatively, lipids and peptides are mixed in a volatile organic solvent with or without a small amount of water. The volatile solvent is evaporated under a stream of nitrogen or argon, in a vacuum oven, or by rotary evaporation either before or after addition of an aqueous solvent.

The mixture of lipid and peptide prepared by one of the methods described above is incubated for up to 2 hours, preferably at 35-50°C with sonic irradiation. The mixture may then be dialyzed, filtered, or chromatographed to replace the aqueous medium with a pharmaceutically acceptable medium, although this is not necessary. In some cases, efficacy is improved by separating unreacted lipid or peptide from associated lipid and peptide by ultracentrifugation, filtration, or chromatography. The mixture may then be lyophilized or aerosolized.

The polypeptide-phospholipid complexes of this invention can be used in the treatment of neonatal respiratory distress syndrome, a physiological condition which results from the inability of the lungs of premature infants to produce pulmonary surfactant. The complexes of this invention when administered to RDS patients, act as synthetic pulmonary surfactants and either replace the natural, missing surfactant or augment the lack of sufficient natural surfactant. Treatment is continued until the infant's lungs produce a sufficient amount of natural, pulmonary surfactant so as to render further treatment unnecessary.

The preparations are preferably those suitable for endotracheal administration, that is as a liquid suspension, a dry powder, or an aerosol. For a liquid suspension, the dry mixture or the mixture in aqueous suspension is mixed with suitable agents, such as water, saline solutions, dextrose, and glycerol to produce a pharmaceutically effective composition. Preferred liquid suspensions will contain 0.8 to 1.0 weight per cent of sodium chloride and will be 1 - 20 mM in calcium ion. The preparation is then filter sterilized. In general, the preparation comprises 1 to 100 mg of DPPC per ml and is administered at a dose of 0.2 to 5 ml/kg. To prepare a dry mixture, the aqueous suspension is lyophilized. The aerosol is prepared from a finely divided dry powder suspended in a propellant, such as lower alkanes and fluorinated alkanes, such as Freon. The aerosol is stored in a pressurized container.

The surfactant is administered, as appropriate to the dosage form, by endotracheal tube, by aerosol administration, or by nebulization of the suspension or dry mixture into the inspired gas. The surfactant is administered in one or multiple doses of 10 to 200 mg/kg. The preferred method of administration is as a suspension of peptide and lipid in physiological saline solution at a concentration of 5-10 mg of surfactant per ml through an endotracheal tube, achieving a dose of 50-100 mg/kg.

## EXAMPLE 1

Preparation of DPPC Complex of H-Ser-Ser-Ala-Asp-Trp-Leu-Lys-Ala-Phe-Tyr-Asp-Lys-Val-Ala-Glu-Lys-Leu-Lys-Glu-Ala-Phe-Ser-Ser-Ser-OH (Peptide 1)

Peptide 1 was prepared by solid-phase synthesis. DPPC (25 mg) in 1 ml of chloroform was dried under a stream of nitrogen and lyophilized to remove traces of organic solvent. To the dry lipid mixture was added 3 ml of water. The preparation was incubated for 1 hour at 45°C. Then, 0.5 mg of dry Peptide I was added to the aqueous preparation. The preparation was sonicated in a bath ultrasonicator at 45°C for 2 hours. The resulting lipid-peptide mixture was lyophilized and stored at 4°C for up to one month. Prior to testing, 9 ml of 0.9% NaCl, 20 mM HEPES buffer, pH 7.40 was added. The preparation was incubated for 1 hour at 45°C with periodic mixing. The preparation has a translucent white appearance and is somewhat less turbid than DPPC alone.

## EXAMPLE 2

Preparation of DPPC Complex of Suc-Lys-Leu-Leu-Glu-Trp-Leu-Lys-Glu-Leu-Leu-NH₂ (Peptide 2)

Peptide 2 was prepared by solid-phase synthesis and mixed with DPPC as described under Example I except that the final suspending buffer contains 5 mM CaCl₂ in addition to 0.9% NaCl, 20 mM HEPES buffer, pH 7.40.

## EXAMPLE 3

Preparation of DPPC Complex of Suc-Leu-Leu-Glu-Lys-Leu-Leu-Glu-Trp-Leu-Lys-NH₂ (Peptide 3)

In a like manner, the Peptide 3 was prepared and its complex with DPPC was formed.

In a like manner but using a mixture of 25 weight per cent DUPG and 75 weight per cent DPPC gives a DUPG/DPPC complex of peptide 2.

## EXAMPLE 4

Preparation of DPPC Complex of Suc-Lys-Leu-Lys-Glu-Leu-Leu-Glu-Lys-Leu-Leu-Glu-Trp-Leu-Lys-NH$_2$ (Peptide 4)

In a like manner, the Peptide 4 was prepared and its complex with DPPC was formed.

## EXAMPLE 5

Suc-Leu-Leu-Glu-Lys-Leu-Leu-Glu-Lys-Leu-Lys-NH$_2$

In a like manner, the Peptide 4 was prepared and its complex with DPPC was formed.

## EXAMPLE 6

Suc-Leu-Leu-Glu-Lys-Leu-Leu-Glu-Phe-Leu-Lys-NH$_2$

In a like manner, the Peptide 4 was prepared and its complex with DPPC was formed.

## EXAMPLE 7

Suc-Leu-Leu-Glu-Lys-Leu-Leu-Glu-Ala-Leu-Lys-NH$_2$

In a like manner, the Peptide 4 was prepared and its complex with DPPC was formed.

## EXAMPLE 8

Suc-Lys-Leu-Leu-Glu-Lys-Leu-Lys-Glu-Leu-Leu-NH$_2$

In a like manner, the Peptide 4 was prepared and its complex with DPPC was formed.

## EXAMPLE 9

Suc-Lys-Leu-Lys-Glu-Leu-Leu-Glu-Lys-Leu-Leu-Glu-Trp(For)-Leu-Lys-NH$_2$

In a like manner, the Peptide 4 was prepared and its complex with DPPC was formed.

## EXAMPLE 10

H-Ser-Ser-Ala-Asp-Trp(For)-Leu-Lys-Ala-Phe-Tyr-Asp-Lys-Val-Ala-Glu-Lys-Leu-Lys-Glu-Ala-Phe-Ser-Ser-Ser-OH

In a like manner, the Peptide 4 was prepared and its complex with DPPC was formed.

TABLE 1

| PHYSICAL PROPERTIES AND AMINO ACID ANALYSIS OF PEPTIDES 1 - 10 | | | | | | | | | | | |
|---|---|---|---|---|---|---|---|---|---|---|---|
| # | Amino Acid Analysis | | | | | | | | | FAB-MS $(M+H)^+$ ± 1m.u | $E_{280}$ |
| | B | S | Z | A | V | L | F | Y | K | | |
| 1 | 1.99 | 4.94 | 2.07 | 3.90 | 1.03 | 2.03 | 2.04 | 1.04 | 3.98 | 2707 | 7040 |
| 2 | | | 2.03 | | | 4.98 | | | 2.00 | 1383 | 5924 |
| 3 | | | 1.97 | | | 5.09 | | | 1.94 | 1384 | 8218 |
| 4 | | 3.06 | | | | 6.01 | | | ·3.93 | 1883 | 6857 |
| 5 | 2.02 | | | | | 5.10 | | | 2.88 | 1326 | -- |
| 6 | 2.08 | | | | | 5.00 | 0.96 | | 1.96 | 1345 | -- |
| 7 | 2.06 | | | 0.99 | | 4.98 | | | 1.96 | 1268 | -- |
| 8 | 2.07 | | | | | 4.99 | | | 2.94 | 1326 | -- |
| 9 | 3.13 | | | | | 6.00 | | | 3.89 | 1909 | -- |
| 10 | 2.03 | 4.53 | 1.53 | 4.08 | 0.95 | 2.02 | 2.00 | 0.98 | 3.95 | 1734 | -- |

## EXAMPLE 11

Pulmonary Surfactant Evaluation of Peptides 1 and 2

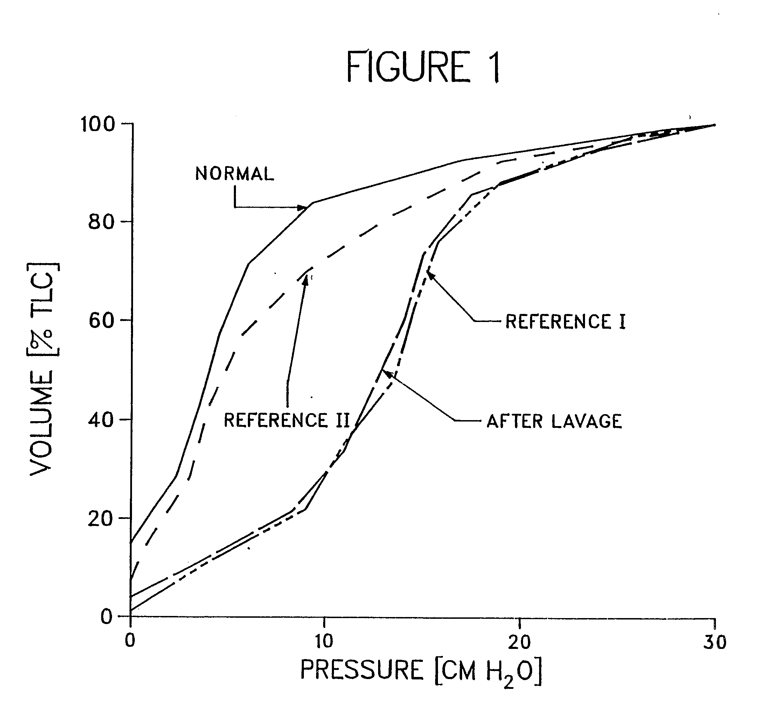

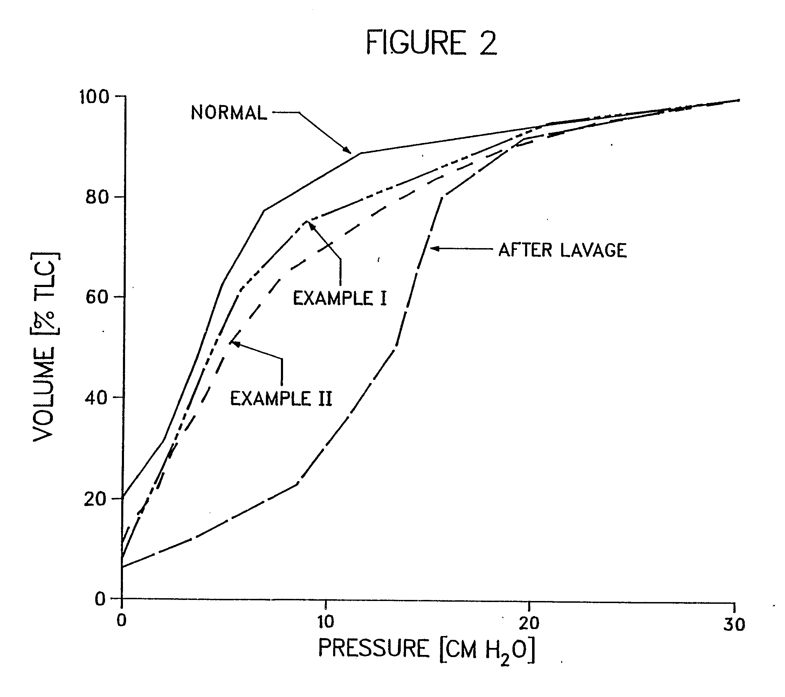

The synthetic surfactant preparations were tested in the adult rat lung model (Ikegami, et al., (1979) Pediatr. Res. 13, 777-780). Reference I and Reference II, consisting of DPPC, alone and dog lung surfactant, respectively, were employed as reference standards. Three ml of the preparations from each of examples 1 and 2 were tested in an adult rat lung model. The preparations of Examples 1 and 2 nearly fully restore the compliance of the rat lung. Results are illustrated in Figures 1 and 2.

## Reference I.

Reference 1 was prepared as follows. DPPC was purchased from Avanti Polar Lipids. 25 mg of DPPC in chloroform was dried under a stream of nitrogen and lyophilized to remove traces of organic solvent. To the dry lipid mixture was added 3 ml of water. The preparation was incubated at 45°C for 1 hour with ultrasonication in a bath sonicator. The lipid was lyophilized, stored at 4°C for up to one week and mixed with 9 ml of 0.9% NaCl, 20 mM HEPES buffer, pH 7.40. After periodic mixing during incubation at 45°C for 1 hour Reference I was tested in the adult rat lung model (Fig. 1a). Reference I has no effect on improving the compliance of the rat lung and is ineffective as a lung surfactant.

### Reference II.

Reference II was prepared as follows. Dog lung surfactant was prepared by methods familiar to those in the art. Reference II restores the lung capacity at 5 cm water to >75% of the fully sufficient rat lung.

## Claims

1. A polypeptide of the formula
X-Y-Z-Y$'$-Q
wherein X is hydrogen, an amino acid, dipeptide, or tripeptide, or an aliphatic, aromatic, or cyclic organic acid having from 1 to 10 carbon atoms;
Y and Y$'$ are each independently a bond or -(Ser)$_n$-where n is an integer of from 1 to 3;
Q is an hydroxy amino, alkylamino or alkoxy group;
Z is a peptide residue of from 8 to 25 amino acids consisting of a fragment of the oligomer having the sequence
-A$_1$-A$_2$-A$_3$-A$_4$-A$_5$-A$_6$-A$_7$-A$_8$-A$_9$-A$_{10}$-A$_{11}$-A$_1'$-A$_2'$-A$_3'$-A$_4'$-A$_5'$-A$_6'$-A$_7'$-A$_8'$-A$_9'$-A$_{10}'$-A$_{11}'$-A$_1''$-A$_2''$-A$_3''$-A$_4''$-A$_5''$-A$_6''$-A$_7''$-A$_8''$-A$_9''$-A$_{10}''$-A$_{11}''$-A$_1'''$-A$_2'''$
and which may begin with any one of the amino acid residues A$_1$ - A$_{11}$
wherein
A$_1$, A$_1'$, A$_1''$, A$_1'''$, A$_4$, A$_4'$, A$_4''$, A$_8$, A$_8'$, and A$_8''$ are each independently selected from the group of hydrophilic amino acids consisting of Glu, Asp, Ala, Gln, Asn, Gly, Ser, Thr, Lys, Arg, Orn, and hArg;
A$_2$, A$_2'$, A$_2''$, A$_2'''$, A$_3$, A$_3'$, A$_3''$, A$_6$, A$_6'$, A$_6''$, A$_7$, A$_7'$, A$_7''$, A$_{10}$, A$_{10}'$, and A$_{10}''$ are each independently selected from the group of lipophilic amino acids consisting of Leu, Nle, Met, Ala, Val, Phe, Nva, Ile and Tyr;
A$_5$, A$_5'$, A$_5''$, A$_{11}$, A$_{11}'$, and A$_{11}''$ are each independently selected from the group of basic amino acids consisting of Lys, Orn, Arg, hArg;
A$_9$, A$_9'$, A$_9''$ are each independently selected from the group of lipophilic neutral or basic amino acids consisting of Leu, Nle, Met, Ala, Val, Phe, Nva, Ile, Tyr, Thr, Ser, Gln, Asn, Gly, Lys, Arg, hArg, Trp, Orn, Trp(For),
or a pharmaceutically acceptable salt thereof, with the proviso that Z cannot be
-Glu-Trp-Leu-Lys-Ala-Phe-Tyr-Glu-Lys-Val-Leu-Glu-Lys-Leu-Lys-Glu-Leu-Phe-;
-Asp-Trp-Leu-Lys-Ala-Phe-Tyr-Asp-Lys-Val-Ala-Glu-Lys-Leu-Lys-Glu-Ala-Phe-;
-Ala-Asp-Trp-Leu-Lys-Ala-Phe-Tyr-Asp-Lys-Val-Ala-Glu-Lys-Leu-Lys-Glu-Na-Phe-; or
-Ala-Asp-Trp-Leu-Lys-Ala-Phe-Tyr-Ser-Lys-Val-Ala-Glu-Ala-Leu-Lys-Glu-Ala-Phe-.
2. The polypeptide of claim 1 wherein at least one of A$_1$, A$_1'$, A$_1''$, or A$_1'''$ is a Glu.
3. The polypeptide of claim 1 or 2 wherein at least one of A$_4$, A$_4'$, or A$_4''$ is a Glu.
4. The polypeptide of any one of claims 1 to 3 wherein at least one of A$_8$, A$_8'$, or A$_8''$ is a Glu.
5. The polypeptide of any one of claims 1 to 4 wherein at least one of A$_2$, A$_2'$, A$_2''$, or A$_2'''$ is a Leu.
6. The polypeptide of any one of claims 1 to 5 wherein at least one of A$_3$, A$_3'$, or A$_3''$ is a Leu.
7. The polypeptide of any one of claims 1 to 6 wherein at least one of A$_6$, A$_6'$, or A$_6''$ is a Leu.
8. The polypeptide of any one of claims 1 to 7 wherein at least one of A$_7$, A$_7'$, or A$_7''$ is a Leu.
9. The polypeptide of any one of claims 1 to 8 wherein at least one of A$_{10}$, A$_{10}'$, or A$_{10}''$ is a Leu.
10. The polypeptide of any one of claims 1 to 9 wherein at least one of A$_5$, A$_5'$, or A$_5''$ is a Lys.
11. The polypeptide of any one of claims 1 to 10 wherein at least one of A$_9$, A$_9'$, or A$_9''$ is an Ala.
12. The polypeptide of any one of claims 1 to 11 wherein at least one of A$_{11}$, A$_{11}'$, or A$_{11}''$ is a Lys.
13. The polypeptide of any one of claims 1 to 12 wherein Q is an amino group.
14. The polypeptide of claim 1 which is H-Ser-Ser-Ala-Asp-Trp-Leu-Lys-Ala-Phe-Tyr-Asp-Lys-Val-Ala-Glu-Lys-Leu-Lys-Glu-Ala-Phe-Ser-Ser-Ser-OH.
15. The polypeptide of claim 1 which is Suc-Lys-Leu-Leu-Glu-Trp-Leu-Lys-Glu-Leu-Leu-NH$_2$.
16. The polypeptide of claim 1 which is Suc-Leu-Leu-Glu-Lys-Leu-Leu-Glu-Trp-Leu-Lys-NH$_2$.
17. The polypeptide of claim 1 which is Suc-Lys-Leu-Lys-Glu-Leu-Leu-Glu-Lys-Leu-Leu-Glu-Trp-Leu-Lys-NH$_2$.
18. The polypeptide of claim 1 which is Suc-Leu-Leu-Glu-Lys-Leu-Leu-Glu-Lys-Leu-Lys-NH$_2$.
19. The polypeptide of claim 1 which is Suc-Leu-Leu-Glu-Lys-Leu-Leu-Glu-Phe-Leu-Lys-NH$_2$.

20. The polypeptide of claim 1 which is Suc-Leu-Leu-Glu-Lys-Leu-Leu-Glu-Ala-Leu-Lys-NH$_2$.

21. The polypeptide of claim 1 which is Suc-Lys-Leu-Leu-Glu-Lys-Leu-Lys-Glu-Leu-Leu-NH$_2$.

22. The polypeptide of claim 1 which is Suc-Lys-Leu-Lys-Glu-Leu-Leu-Glu-Lys-Leu-Leu-Glu-Trp(For)-Leu-Lys-NH$_2$.

23. The polypeptide of claim 1 which is H-Ser-Ser-Ala-Asp-Trp(For)-Leu-Lys-Ala-Phe-Tyr-Asp-Lys-Val-Ala-Glu-Lys-Leu-Lys-Glu-Ala-Phe-Ser-Ser-Ser-OH.

24. A complex of a polypeptide according to any one of claims 1 to 23 (including the polypeptides of the disclaimer of claim 1) or of a pharmaceutically acceptable salt thereof with the lipid DPPC, PC, CL, PG, PS, FA, or TG or with a mixture thereof.

25. The complex of claim 24 wherein DPPC comprises the major component of the lipid.

26. The complex of claim 24 wherein the lipid is a mixture of DPPC and PG.

27. The complex of any one of claims 24 to 26 wherein the lipid consists of from about 85-100% DPPC and from about 0 - 15% PG.

28. A process for preparing a polypeptide according to any one of claims 1 to 23 or a polypeptide contained in the complex of claim 24 which comprises a solid phase sequential and/or block synthesis, the cloning of a corresponding gene or combinations thereof.

29. A process according to claim 28, wherein the solid phase sequential or block synthesis comprises binding a suitably protected amino acid corresponding to the amino terminal amino acid to an activated resin support, and subsequently binding the other alpha amino protected amino acids in order from the amino to carboxy terminal end to the terminal amino group of the growing peptidic chain which has meanwhile been exposed by removing its amino protecting group.

30. A polypeptide according to any one of claims 1 to 23 (including the polypeptides of the disclaimer of claim 1) or a complex according to any one of claims 24 to 27 for use as a pharmaceutically active substance.

31. A polypeptide according to any one of claims 1 to 23 (including the polypeptides of the disclaimer of claim 1) or a complex according to any one of claims 24 to 27 for use as a pharmaceutically active substance for the treatment of the respiratory distress syndrome.

32. A pharmaceutical composition containing a polypeptide according to any one of claims 1 to 23 (including the polypeptides of the disclaimer of claim 1) or a complex according to any one of claims 24 to 27.

33. A pharmaceutical composition for the treatment of the respiratory distress syndrome containing a polypeptide according to any one of claims 1 to 23 (including the polypeptides of the disclaimer of claim 1) or a complex according to any one of claims 24 to 27.

Claims for the following Contracting State: GR

1. A polypeptide of the formula

**X-Y-Z-Y$'$-Q**

wherein X is hydrogen, an amino acid, dipeptide, or tripeptide, or an aliphatic, aromatic, or cyclic organic acid having from 1 to 10 carbon atoms;

Y and Y$'$ are each independently a bond or -(Ser)$_n$-where n is an integer of from 1 to 3;

Q is an hydroxy amino, alkylamino or alkoxy group;

Z is a peptide residue of from 8 to 25 amino acids consisting of a fragment of the oligomer having the sequence

-A$_1$-A$_2$-A$_3$-A$_4$-A$_5$-A$_6$-A$_7$-A$_8$-A$_9$-A$_{10}$-A$_{11}$-A$_1'$-A$_2'$-A$_3'$-A$_4'$-A$_5'$-A$_6'$-A$_7'$-A$_8'$-A$_9'$-A$_{10}'$-A$_{11}'$-A$_1''$-A$_2''$-A$_3''$-A$_4''$-A$_5''$-A$_6''$-A$_7''$-A$_8''$-A$_9''$-A$_{10}''$-A$_{11}''$-A$_1'''$-A$_2'''$-

and which may begin with any one of the amino acid residues A$_1$ - A$_{11}$

wherein

A$_1$, A$_1'$, A$_1''$, A$_1'''$, A$_4$, A$_4'$, A$_4''$, A$_8$, A$_8'$, and A$_8''$ are each independently selected from the group of hydrophilic amino acids consisting of Glu, Asp, Ala, Gln, Asn, Gly, Ser, Thr, Lys, Arg, Orn, and hArg;

A$_2$, A$_2'$, A$_2''$, A$_2'''$, A$_3$, A$_3'$, A$_3''$, A$_6$, A$_6'$, A$_6''$, A$_7$, A$_7'$, A$_7''$, A$_{10}$, A$_{10}'$, and A$_{10}''$ are each independently selected from the group of lipophilic amino acids consisting of Leu, Nle, Met, Ala, Val, Phe, Nva, Ile and Tyr;

A$_5$, A$_5'$, A$_5''$, A$_{11}$, A$_{11}'$, and A$_{11}''$ are each independently selected from the group of basic amino acids consisting of Lys, Orn, Arg, hArg;

A$_9$, A$_9'$, A$_9''$ are each independently selected from the group of lipophilic neutral or basic amino acids consisting of Leu, Nle, Met, Ala, Val, Phe, Nva, Ile, Tyr, Thr, Ser, Gln, Asn, Gly, Lys, Arg, hArg, Trp, Orn, Trp(For),

or a pharmaceutically acceptable salt thereof, with the proviso that Z cannot be
-Glu-Trp-Leu-Lys-Ala-Phe-Tyr-Glu-Lys-Val-Leu-Glu-Lys-Leu-Lys-Glu-Leu-Phe-;
-Asp-Trp-Leu-Lys-Ala-Phe-Tyr-Asp-Lys-Val-Ala-Glu-Lys-Leu-Lys-Glu-Ala-Phe-;
-Ala-Asp-Trp-Leu-Lys-Ala-Phe-Tyr-Asp-Lys-Val-Ala-Glu-Lys-Leu-Lys-Glu-Na-Phe-; or
-Ala-Asp-Trp-Leu-Lys-Ala-Phe-Tyr-Ser-Lys-Val-Ala-Glu-Ala-Leu-Lys-Glu-Ala-Phe-.

2. The polypeptide of claim 1 wherein at least one of $A_1$, $A_1{}'$, $A_1{}''$, or $A_1{}'''$ is a Glu.

3. The polypeptide of claim 1 or 2 wherein at least one of $A_4$, $A_4{}'$, or $A_4{}''$ is a Glu.

4. The polypeptide of any one of claims 1 to 3 wherein at least one of $A_8$, $A_8{}'$, or $A_8{}''$ is a Glu.

5. The polypeptide of any one of claims 1 to 4 wherein at least one of $A_2$, $A_2{}'$, $A_2{}''$, or $A_2{}'''$ is a Leu.

6. The polypeptide of any one of claims 1 to 5 wherein at least one of $A_3$, $A_3{}'$, or $A_3{}''$ is a Leu.

7. The polypeptide of any one of claims 1 to 6 wherein at least one of $A_6$, $A_6{}'$, or $A_6{}''$ is a Leu.

8. The polypeptide of any one of claims 1 to 7 wherein at least one of $A_7$, $A_7{}'$, or $A_7{}''$ is a Leu.

9. The polypeptide of any one of claims 1 to 8 wherein at least one of $A_{10}$, $A_{10}{}'$, or $A_{10}{}''$ is a Leu.

10. The polypeptide of any one of claims 1 to 9 wherein at least one of $A_5$, $A_5{}'$, or $A_5{}''$ is a Lys.

11. The polypeptide of any one of claims 1 to 10 wherein at least one of $A_9$, $A_9{}'$, or $A_9{}''$ is an Ala.

12. The polypeptide of any one of claims 1 to 11 wherein at least one of $A_{11}$, $A_{11}{}'$, or $A_{11}{}''$ is a Lys.

13. The polypeptide of any one of claims 1 to 12 wherein Q is an amino group.

14. The polypeptide of claim 1 which is H-Ser-Ser-Ala-Asp-Trp-Leu-Lys-Ala-Phe-Tyr-Asp-Lys-Val-Ala-Glu-Lys-Leu-Lys-Glu-Ala-Phe-Ser-Ser-Ser-OH.

15. The polypeptide of claim 1 which is Suc-Lys-Leu-Leu-Glu-Trp-Leu-Lys-Glu-Leu-Leu-NH$_2$.

16. The polypeptide of claim 1 which is Suc-Leu-Leu-Glu-Lys-Leu-Leu-Glu-Trp-Leu-Lys-NH$_2$.

17. The polypeptide of claim 1 which is Suc-Lys-Leu-Lys-Glu-Leu-Leu-Glu-Lys-Leu-Leu-Glu-Trp-Leu-Lys-NH$_2$.

18. The polypeptide of claim 1 which is Suc-Leu-Leu-Glu-Lys-Leu-Leu-Glu-Lys-Leu-Lys-NH$_2$.

19. The polypeptide of claim 1 which is Suc-Leu-Leu-Glu-Lys-Leu-Leu-Glu-Phe-Leu-Lys-NH$_2$.

20. The polypeptide of claim 1 which is Suc-Leu-Leu-Glu-Lys-Leu-Leu-Glu-Ala-Leu-Lys-NH$_2$.

21. The polypeptide of claim 1 which is Suc-Lys-Leu-Leu-Glu-Lys-Leu-Lys-Glu-Leu-Leu-NH$_2$.

23. The polypeptide of claim 1 which is Suc-Lys-Leu-Lys-Glu-Leu-Leu-Glu-Lys-Leu-Leu-Glu-Trp(For)-Leu-Lys-NH$_2$.

24. The polypeptide of claim 1 which is H-Ser-Ser-Ala-Asp-Trp(For)-Leu-Lys-Ala-Phe-Tyr-Asp-Lys-Val-Ala-Glu-Lys-Leu-Lys-Glu-Ala-Phe-Ser-Ser-Ser-OH.

25. A complex of a polypeptide according to any one of claims 1 to 23 (including the polypeptides of the disclaimer of claim 1) or of a pharmaceutically acceptable salt thereof with the lipid DPPC, PC, CL, PG, PS, FA, or TG or with a mixture thereof.

26. The complex of claim 24 wherein DPPC comprises the major component of the lipid.

27. The complex of claim 24 wherein the lipid is a mixture of DPPC and PG.

27. The complex of any one of claims 24 to 26 wherein the lipid consists of from about 85 - 100% DPPC and from about 0 - 15% PG.

28. A process for preparing a polypeptide according to any one of claims 1 to 23 or a polypeptide contained in the complex of claim 24 which comprises a solid phase sequential and/or block synthesis, the cloning of a corresponding gene or combinations thereof.

29. A process according to claim 28, wherein the solid phase sequential or block synthesis comprises binding a suitably protected amino acid corresponding to the amino terminal amino acid to an activated resin support, and subsequently binding the other alpha amino protected amino acids in order from the amino to carboxy terminal end to the terminal amino group of the growing peptidic chain which has meanwhile been exposed by removing its amino protecting group.

30. A polypeptide according to any one of claims 1 to 23 (including the polypeptides of the disclaimer of claim 1) or a complex according to any one of claims 24 to 27 for use as a pharmaceutically active substance.

31. A polypeptide according to any one of claims 1 to 23 (including the polypeptides of the disclaimer of claim 1) or a complex according to any one of claims 24 to 27 for use as a pharmaceutically active substance for the treatment of the respiratory distress syndrome.

Claims for the following Contracting State: ES

1. A process for preparing a peptide derivative of the formula

X-Y-Z-Y'-Q

wherein X is hydrogen, an amino acid, dipeptide, or tripeptide, or an aliphatic, aromatic, or cyclic organic acid having from 1 to 10 carbon atoms;

Y and Y$'$ are each independently a bond or -(Ser)$_n$-where n is an integer of from 1 to 3;

Q is an hydroxy amino, alkylamino or alkoxy group;

Z is a peptide residue of from 8 to 25 amino acids consisting of a fragment of the oligomer having the sequence

-A$_1$-A$_2$-A$_3$-A$_4$-A$_5$-A$_6$-A$_7$-A$_8$-A$_9$-A$_{10}$-A$_{11}$-A$_1'$-A$_2'$-A$_3'$-A$_4'$-A$_5'$-A$_6'$-A$_7'$-A$_8'$-A$_9'$-A$_{10}'$-A$_{11}'$-A$_1''$-A$_2''$-A$_3''$-A$_4''$-A$_5''$-A$_6''$-A$_7''$-A$_8''$-A$_9''$-A$_{10}''$-A$_{11}''$-A$_1'''$-A$_2'''$-

and which may begin with any one of the amino acid residues A$_1$ - A$_{11}$

wherein

A$_1$, A$_1'$, A$_1''$, A$_1'''$, A$_4$, A$_4'$, A$_4''$, A$_8$, A$_8'$, and A$_8''$ are each independently selected from the group of hydrophilic amino acids consisting of Glu, Asp, Ala, Gln, Asn, Gly, Ser, Thr, Lys, Arg, Orn, and hArg;

A$_2$, A$_2'$, A$_2''$, A$_2'''$, A$_3$, A$_3'$, A$_3''$, A$_6$, A$_6'$, A$_6''$, A$_7$, A$_7'$, A$_7''$, A$_{10}$, A$_{10}'$, and A$_{10}''$ are each independently selected from the group of lipophilic amino acids consisting of Leu, Nle, Met, Ala, Val, Phe, Nva, Ile and Tyr;

A$_5$, A$_5'$, A$_5''$, A$_{11}$, A$_{11}'$, and A$_{11}''$ are each independently selected from the group of basic amino acids consisting of Lys, Orn, Arg, hArg;

A$_9$, A$_9'$, A$_9''$ are each independently selected from the group of lipophilic neutral or basic amino acids consisting of Leu, Nle, Met, Ala, Val, Phe, Nva, Ile, Tyr, Thr, Ser, Gln, Asn, Gly, Lys, Arg, hArg, Trp, Orn, Trp(For),

or a pharmaceutically acceptable salt thereof, with the proviso that Z cannot be

-Glu-Trp-Leu-Lys-Ala-Phe-Tyr-Glu-Lys-Val-Leu-Glu-Lys-Leu-Lys-Glu-Leu-Phe-;

-Asp-Trp-Leu-Lys-Ala-Phe-Tyr-Asp-Lys-Val-Ala-Glu-Lys-Leu-Lys-Glu-Ala-Phe-;

-Ala-Asp-Trp-Leu-Lys-Ala-Phe-Tyr-Asp-Lys-Val-Ala-Glu-Lys-Leu-Lys-Glu-Na-Phe-; or

-Ala-Asp-Trp-Leu-Lys-Ala-Phe-Tyr-Ser-Lys-Val-Ala-Glu-Ala-Leu-Lys-Glu-Ala-Phe-

by solid phase sequential and block synthesis, gene cloning or combinations thereof.

2. The process according to claim 1 wherein the solid phase sequential or block synthesis comprises binding a suitably protected amino acid corresponding to the amino terminal amino acid to an activated resin support, and subsequently binding the other alpha amino protected amino acids in order from the amino to carboxy terminal end to the terminal amino group of the growing peptidic chain which has meanwhile been exposed by removing its amino protecting group.

3. The process according to claims 1 or 2 wherein at least one of A$_1$, A$_1'$, A$_1''$, or A$_1'''$ is a Glu.

4. The process according to any one of claims 1 to 3 wherein at least one of A$_4$, A$_4'$, or A$_4''$ is a Glu.

5. The process according to any one of claims 1 to 4 wherein at least one of A$_8$, A$_8'$, or A$_8''$ is a Glu.

6. The process according to any one of claims 1 to 5 wherein at least one of A$_2$, A$_2'$, A$_2''$, or A$_2'''$ is a Leu.

7. The process according to any one of claims 1 to 6 wherein at least one of A$_3$, A$_3'$, or A$_3''$ is a Leu.

8. The process according to any one of claims 1 to 7 wherein at least one of A$_6$, A$_6'$, or A$_6''$ is a Leu.

9. The process according to any one of claims 1 to 8 wherein at least one of A$_7$, A$_7'$, or A$_7''$ is a Leu.

10. The process according to any one of claims 1 to 9 wherein at least one of A$_{10}$, A$_{10}'$, or A$_{10}''$ is a Leu.

11. The process according to any one of claims 1 to 10 wherein at least one of A$_5$, A$_5'$, or A$_5''$ is a Lys.

12. The process according to any one of claims 1 to 11 wherein at least one of A$_9$, A$_9'$, or A$_9''$ is an Ala.

13. The process according to any one of claims 1 to 12 wherein at least one of A$_{11}$, A$_{11}'$, or A$_{11}''$ is a Lys.

14. The process according to any one of claims 1 to 13 wherein Q is an amino group.

15. The process according to claims 1 or 2 for preparing a peptide derivative which is H-Ser-Ser-Ala-Asp-Trp-Leu-Lys-Ala-Phe-Tyr-Asp-Lys-Val-Ala-Glu-Lys-Leu-Lys-Glu-Ala-Phe-Ser-Ser-Ser-OH.

16. The process according to claims 1 or 2 for preparing a peptide derivative which is Suc-Lys-Leu-Leu-Glu-Trp-Leu-Lys-Glu-Leu-Leu-NH$_2$.

17. The process according to claims 1 or 2 for preparing a peptide derivative which is Suc-Leu-Leu-Glu-Lys-Leu-Leu-Glu-Trp-Leu-Lys-NH$_2$.

18. The process according to claims 1 or 2 for preparing a peptide derivative which is Suc-Lys-Leu-Lys-Glu-Leu-Leu-Glu-Lys-Leu-Leu-Glu-Trp-Leu-Lys-NH$_2$.

19. The process according to claims 1 or 2 for preparing a peptide derivative which is Suc-Leu-Leu-Glu-Lys-Leu-Leu-Glu-Lys-Leu-Lys-NH$_2$.

20. The process according to claims 1 or 2 for preparing a peptide derivative which is Suc-Leu-Leu-Glu-Lys-Leu-Leu-Glu-Phe-Leu-Lys-NH$_2$.

21. The process according to claims 1 or 2 for preparing a peptide derivative which is Suc-Leu-Leu-Glu-

EP 0 348 967 A2

Lys-Leu-Leu-Glu-Ala-Leu-Lys-NH$_2$.

22. The process according to claims 1 or 2 for preparing a peptide derivative which is Suc-Lys-Leu-Leu-Glu-Lys-Leu-Lys-Glu-Leu-Leu-NH$_2$.

23. The process according to claims 1 or 2 for preparing a peptide derivative which is Suc-Lys-Leu-Lys-Glu-Leu-Leu-Glu-Lys-Leu-Leu-Glu-Trp(For)-Leu-Lys-NH$_2$.

24. The process according to claims 1 or 2 for preparing a peptide derivative which is H-Ser-Ser-Ala-Asp-Trp(For)-Leu-Lys-Ala-Phe-Tyr-Asp-Lys-Val-Ala-Glu-Lys-Leu-Lys-Glu-Ala-Phe-Ser-Ser-Ser-OH.

25. A process according to any one of claims 1 to 24 which additionally comprises the formation of a complex of the obtained polypeptide (including the polypeptides of the disclaimer of claim 1) with the lipid DPPC, PC, CL, PG, PS, FA, or TG or with a mixture thereof.

26. Use of a polypeptide obtainable according to a process of any one of claims 1 to 24 (including the polypeptides of the disclaimer of claim 1) or of a complex obtainable according to claim 25 for the preparation of a pharmaceutical composition.

27. Use of a polypeptide obtainable according to a process of any one of claims 1 to 24 (including the polypeptides of the disclaimer of claim 1) or of a complex obtainable according to claim 25 for the preparation of a pharmaceutical composition for the treatment of the respiratory distress syndrome.

14

# FIGURE 1

EP 0 348 967 A2

## FIGURE 2

EP 0 348 967 A2